# EUROPEAN PATENT APPLICATION

(11) **EP 2 133 434 A1**
(43) Date of publication of application: **16.12.2009**
(21) Application number: 08305256.3
(22) Date of filing: 12.06.2008
(51) Int. Cl.: C12Q 1/68

(54) **Method for the detection of target nucleic acid**

(71) Applicant: Institut National de la Santé et de la Recherche Médicale (INSERM), 75013 Paris (FR)
(72) Inventor: The designation of the inventor has not yet been filed
(74) Representative: Warcoin, Jacques

(57) **Abstract**

The present invention is directed to a method for detecting at least one target nucleic acid X in a sample, comprising the steps of: a) contacting said sample with a polymerase, natural dNTPs, at least one oligonucleotide probe comprising a first non-natural nucleotide located at its 5' end, and a first label, at least one couple of two oligonucleotide primers complementary to 5' and 3' nucleotidic regions respectively of said at least one target nucleic acid X, and at least one reporter comprising at least a second label coupled to a second non-natural nucleotide, which second non-natural nucleotide is complementary to said first non-natural nucleotide; b) conducting a polymerase chain reaction under conditions wherein said at least one target nucleic acid X, if present in the sample, is amplified using said couple of primers and said oligonucleotide probe for generating oligonucleotide amplification product having (i) a double-stranded region and (ii) a single-stranded region that comprises the first non-natural nucleotide; c) incorporating the second non-natural nucleotide of the reporter into the oligonucleotide amplification product opposite to the first non natural nucleotide of the single stranded region; d) detecting the incorporation of the reporter; **characterised in that** i) one of said primers of said at least one couple of primers comprises a first oligonucleotide tag sequence A in its 5' region; and ii) said at least one oligonucleotide probe consists in said first oligonucleotide tag sequence A, said first non-natural nucleotide located at its 5' end, and said first label.

SEQUENCE LISTING

<110>  Institut national de la santé et de la recherche médicale
       (INSERM)

<120>  IMPROVED METHOD FOR THE DETECTION OF TARGET NUCLEIC ACID

<130>  67310

<160>  13

<170>  PatentIn version 3.3

<210>  1
<211>  20
<212>  DNA
<213>  artificial sequence

<220>
<223>  oligonucleotide tag sequence

<400>  1
gctcgttacg tggttgagac                                                   20


<210>  2
<211>  18
<212>  DNA
<213>  artificial sequence

<220>
<223>  oligonucleotide tag sequence

<400>  2
cacttagcac cacggaca                                                     18


<210>  3
<211>  19
<212>  DNA
<213>  artificial sequence

<220>
<223>  oligonucleotide tag sequence

<400>  3
cgttgtaacg ccccagcac                                                    19


<210>  4
<211>  20
<212>  DNA
<213>  artificial sequence

<220>
<223>  oligonucleotide tag sequence

<400>  4
gcctcttccg gttaccgtgt                                                   20


<210>  5
<211>  20
<212>  DNA
<213>  artificial sequence

<220>
<223>  oligonucleotide tag sequence

<400>  5
aattaaccct cactaaaggg                                                    20


<210>  6
<211>  18
<212>  DNA
<213>  artificial sequence

<220>
<223>  oligonucleotide tag sequence

<400>  6
ctatagtgag tcgtatta                                                      18


<210>  7
<211>  35
<212>  DNA
<213>  artificial sequence

<220>
<223>  Primer 421 with Tag1

<400>  7
aattaaccct cactaaaggg gtggtgaagg aagag                                   35


<210>  8
<211>  19
<212>  DNA
<213>  artificial sequence

<220>
<223>  Primer 422

<400>  8
tctccgtgtc attgtccag                                                     19


<210>  9
<211>  37
<212>  DNA
<213>  artificial sequence

<220>
<223>  Primer with tag2

<400>  9
ctatagtgag tcgtattagt gacaacgtcg agcacag                                 37


<210>  10
<211>  20
<212>  DNA
<213>  artificial sequence

<220>
<223>  Primer 4

<400>  10
tgaatgaact gcaggacgag                                                    20


<210>  11
<211>  2151
<212>  DNA
<213>  Mus musculus

<400> 11
atgggcgaga ccaagatcat ctaccacctg gacggccagg agacgccgta cctggtgaag 60
ctgcccctgc ccgccgagcg cgtcaccttg gcggacttta agggcgttct gcagcggccc 120
agctataagt tcttcttcaa gtctatggac gacgattttg gggtggtgaa ggaagagatc 180
tcggacgaca atgccaagct accctgcttc aatggccggg tggtgtcctg gctggtatct 240
gccgagggct cacacccaga gccggcccct ttctgtgctg acaacccgtc agagctgcca 300
ccttcgatgg aacgcacagg aggcattggg gactctcgac ccccctcctt ccaccctcat 360
gctagtgggg gcagccagga gaatctggac aatgacacgg agaccgactc cttggtgtct 420
gcccaacgag agcggccccg acggagggat ggcccggagc acgcagcccg actcaatgga 480
accacaaagg gtgagcggcg acgggagcca ggcggctatg acagttcatc taccctcatg 540
agcagcgagc tggagactac cagtttcttt gattccgatg aggatgattc caccagcagg 600
ttcagcagct ccacagaaca gagcagtgcc tcacgcctca tgagaagaca caagcggcgg 660
cggcggaagc agaaggtctc ccggattgag cggtcctcgt ctttcagcag catcacagac 720
tccactatgt ctctcaacat catcaccgtc actctcaaca tggaaaagta taacttcttg 780
ggcatctcca ttgtgggcca aagcaatgag cgaggtgatg ggggcatcta cattggctcc 840
atcatgaagg gtggggctgt ggcagctgat ggacgcattg agccaggaga tatgctactg 900
caggtaaatg agatcaactt cgagaacatg agcaacgatg acgcagtccg agtccttcgg 960
gagattgtgc acaagccagg gcccatcact ctgactgtag ccaagtgctg ggacccaagt 1020
cctcgcggct gcttcacatt accccggagc gagcccatcc gtcccattga ccctgcagcc 1080
tgggtctctc acacggcagc catgacaggc accttccccg cctatggcat gagcccctcc 1140
ttaagcacca tcacctctac cagctcctcc atcaccagct ccatccctga tacagagcgc 1200
ctagatgatt tccacctctc catccacagt gacatggcgg ccatcgtaaa agccatggcc 1260
tcccctgagt cagggttgga ggtccgagac cgcatgtggc tcaagattac cattcccaat 1320
gctttcatcg gctcggatgt ggtggactgg ctgtaccaca acgtggaagg gttcaccgac 1380
cgtcgggagg cccgcaagta tgctagcaac ctgctgaaag ctggtttcat ccgccatacc 1440
gtcaacaaga tcacgttctc cgagcagtgc tactacatct ttggcgacct ctgtggtaac 1500
atggccaacc tatcgcttca cgaccatgat ggctccagtg gcgcctctga ccaggacaca 1560
ctggctcctt tgccgcaccc aggagctgcc ccttggccta tggctttccc ttaccagtac 1620
ccaccacctc cgcatcccta caacccacac ccaggcttcc cagaactggg ctacagctac 1680
ggtgggggca gcgccagcag tcagcacagt gaaggcagtc ggagcagtgg ctccaaccga 1740
agtggcagcg accggcgcaa ggagaaggac ccaaaagctg gggactccaa gtcaggtggt 1800
agcggcagcg agtcagacca cactacacgc agcagtctga gggggccacg ggagcgggca 1860
ccaagcgagc gatcaggccc agcggccagc gagcacagcc accgcagtca ccattcgctg 1920
accagcagcc tgcgcagcca ccacacacac ccaagctatg gcccacccgg agtgccccct 1980
ctctatggtc ccccgatgct gatgatgacc ccgccgccgg cagccatggg tcccccagga 2040

gcccctccag ggcgtgacct ggcctcggtg cccccggaac tgacggccag cagacagtcc 2100

ttccgcatgg caatgggaaa ccccagtgag ttctttgtgg atgtgatgtg a 2151

<210> 12
<211> 794
<212> DNA
<213> Trypanosoma brucei brucei

<400> 12
tgattgaaca agatggattg cacgcaggtt ctccggccgc ttgggtggag aggctattcg 60

gctatgactg ggcacaacag acaatcggct gctctgatgc cgccgtgttc cggctgtcag 120

cgcaggggcg cccggttctt tttgtcaaga ccgacctgtc cggtgccctg aatgaactgc 180

aggacgaggc agcgcggcta tcgtggctgg ccacgacggg cgttccttgc gcagctgtgc 240

tcgacgttgt cactgaagcg ggaagggact ggctgctatt gggcgaagtg ccggggcagg 300

atctcctgtc atctcacctt gctcctgccg agaaagtatc catcatggct gatgcaatgc 360

ggcggctgca tacgcttgat ccggctacct gcccattcga ccaccaagcg aaacatcgca 420

tcgagcgagc acgtactcgg atggaagccg gtcttgtcga tcaggatgat ctggacgaag 480

agcatcaggg gctcgcgcca gccgaactgt tcgccaggct caaggcgcgc atgcccgacg 540

gcgaggatct cgtcgtgacc catggcgatg cctgcttgcc gaatatcatg gtggaaaatg 600

gccgcttttc tggattcatc gactgtggcc ggctgggtgt ggcggaccgc tatcaggaca 660

tagcgttggc tacccgtgat attgctgaag agcttggcgg cgaatgggct gaccgcttcc 720

tcgtgcttta cggtatcgcc gctcccgatt cgcagcgcat cgccttctat cgccttcttg 780

acgagttctt ctga 794

<210> 13
<211> 4712
<212> DNA
<213> artificial sequence

<220>
<223> Plasmid pmaxFP-Green-N vector

<400> 13
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacggtacc gcgggcccgg 660
gatccaccgg tcgccaccat ggagagcgac gagagcggcc tgcccgccat ggagatcgag 720
tgccgcatca ccggcaccct gaacggcgtg gagttcgagc tggtgggcgg cggagagggc 780
acccccgagc agggccgcat gaccaacaag atgaagagca ccaaaggcgc cctgaccttc 840
agcccctacc tgctgagcca cgtgatgggc tacggcttct accacttcgg cacctacccc 900
agcggctacg agaacccctt cctgcacgcc atcaacaacg gcggctacac caacacccgc 960
atcgagaagt acgaggacgg cggcgtgctg cacgtgagct tcagctaccg ctacgaggcc 1020
ggccgcgtga tcggcgactt caaggtgatg ggcaccggct tccccgagga cagcgtgatc 1080
ttcaccgaca agatcatccg cagcaacgcc accgtggagc acctgcaccc catgggcgat 1140
aacgatctgg atggcagctt cacccgcacc ttcagcctgc gcgacggcgg ctactacagc 1200
tccgtggtgg acagccacat gcacttcaag agcgccatcc accccagcat cctgcagaac 1260
gggggcccca tgttcgcctt ccgccgcgtg gaggaggatc acagcaacac cgagctgggc 1320
atcgtggagt accagcacgc cttcaagacc ccggatgcag atgccggtga agaataaagc 1380
ggccgcgact ctagatcata atcagccata ccacatttgt agaggtttta cttgctttaa 1440
aaaacctccc acacctcccc ctgaacctga aacataaaat gaatgcaatt gttgttgtta 1500
acttgtttat tgcagcttat aatggttaca aataaagcaa tagcatcaca aatttcacaa 1560
ataaagcatt tttttcactg cattctagtt gtggtttgtc caaactcatc aatgtatctt 1620
aaggcgtaaa ttgtaagcgt taatattttg ttaaaattcg cgttaaattt ttgttaaatc 1680
agctcatttt ttaaccaata ggccgaaatc ggcaaaatcc cttataaatc aaaagaatag 1740
accgagatag ggttgagtgt tgttccagtt tggaacaaga gtccactatt aaagaacgtg 1800
gactccaacg tcaaagggcg aaaaaccgtc tatcagggcg atggcccact acgtgaacca 1860
tcaccctaat caagtttttt ggggtcgagg tgccgtaaag cactaaatcg gaaccctaaa 1920
gggagccccc gatttagagc ttgacgggga aagccggcga acgtggcgag aaaggaaggg 1980
aagaaagcga aaggagcggg cgctagggcg ctggcaagtg tagcggtcac gctgcgcgta 2040
accaccacac ccgccgcgct taatgcgccg ctacagggcg cgtcaggtgg cacttttcgg 2100
ggaaatgtgc gcggaacccc tatttgttta tttttctaaa tacattcaaa tatgtatccg 2160
ctcatgagac aataaccctg ataaatgctt caataatatt gaaaaaggaa gagtcctgag 2220
gcggaaagaa ccagctgtgg aatgtgtgtc agttagggtg tggaaagtcc ccaggctccc 2280
cagcaggcag aagtatgcaa agcatgcatc tcaattagtc agcaaccagg tgtggaaagt 2340
ccccaggctc cccagcaggc agaagtatgc aaagcatgca tctcaattag tcagcaacca 2400
tagtcccgcc cctaactccg cccatcccgc ccctaactcc gcccagttcc gcccattctc 2460
cgccccatgg ctgactaatt ttttttattt atgcagaggc cgaggccgcc tcggcctctg 2520
agctattcca gaagtagtga ggaggctttt ttggaggcct aggcttttgc aaagatcgat 2580
caagagacag gatgaggatc gtttcgcatg attgaacaag atggattgca cgcaggttct 2640
ccggccgctt gggtggagag gctattcggc tatgactggg cacaacagac aatcggctgc 2700

tctgatgccg ccgtgttccg gctgtcagcg caggggcgcc cggttctttt tgtcaagacc 2760
gacctgtccg gtgccctgaa tgaactgcaa gacgaggcag cgcggctatc gtggctggcc 2820
acgacgggcg ttccttgcgc agctgtgctc gacgttgtca ctgaagcggg aagggactgg 2880
ctgctattgg gcgaagtgcc ggggcaggat ctcctgtcat ctcaccttgc tcctgccgag 2940
aaagtatcca tcatggctga tgcaatgcgg cggctgcata cgcttgatcc ggctacctgc 3000
ccattcgacc accaagcgaa acatcgcatc gagcgagcac gtactcggat ggaagccggt 3060
cttgtcgatc aggatgatct ggacgaagag catcaggggc tcgcgccagc cgaactgttc 3120
gccaggctca aggcgagcat gcccgacggc gaggatctcg tcgtgaccca tggcgatgcc 3180
tgcttgccga atatcatggt ggaaaatggc cgcttttctg gattcatcga ctgtggccgg 3240
ctgggtgtgg cggaccgcta tcaggacata gcgttggcta cccgtgatat tgctgaagag 3300
cttggcggcg aatgggctga ccgcttcctc gtgctttacg gtatcgccgc tcccgattcg 3360
cagcgcatcg ccttctatcg ccttcttgac gagttcttct gagcgggact ctggggttcg 3420
aaatgaccga ccaagcgacg cccaacctgc catcacgaga tttcgattcc accgccgcct 3480
tctatgaaag gttgggcttc ggaatcgttt tccgggacgc cggctggatg atcctccagc 3540
gcggggatct catgctggag ttcttcgccc accctagggg gaggctaact gaaacacgga 3600
aggagacaat accggaagga acccgcgcta tgacggcaat aaaaagacag aataaaacgc 3660
acggtgttgg gtcgtttgtt cataaacgcg gggttcggtc ccagggctgg cactctgtcg 3720
ataccccacc gagaccccat tggggccaat acgcccgcgt ttcttccttt tccccacccc 3780
accccccaag ttcgggtgaa ggcccagggc tcgcagccaa cgtcggggcg gcaggccctg 3840
ccatagcctc aggttactca tatatacttt agattgattt aaaacttcat ttttaattta 3900
aaaggatcta ggtgaagatc ctttttgata atctcatgac caaaatccct taacgtgagt 3960
tttcgttcca ctgagcgtca gaccccgtag aaaagatcaa aggatcttct tgagatcctt 4020
tttttctgcg cgtaatctgc tgcttgcaaa caaaaaaacc accgctacca gcggtggttt 4080
gtttgccgga tcaagagcta ccaactcttt ttccgaaggt aactggcttc agcagagcgc 4140
agataccaaa tactgttctt ctagtgtagc cgtagttagg ccaccacttc aagaactctg 4200
tagcaccgcc tacatacctc gctctgctaa tcctgttacc agtggctgct gccagtggcg 4260
ataagtcgtg tcttaccggg ttggactcaa gacgatagtt accggataag gcgcagcggt 4320
cgggctgaac ggggggttcg tgcacacagc ccagcttgga gcgaacgacc tacaccgaac 4380
tgagatacct acagcgtgag ctatgagaaa gcgccacgct tcccgaaggg agaaaggcgg 4440
acaggtatcc ggtaagcggc agggtcggaa caggagagcg cacgagggag cttccagggg 4500
gaaacgcctg gtatctttat agtcctgtcg ggtttcgcca cctctgactt gagcgtcgat 4560
ttttgtgatg ctcgtcaggg gggcggagcc tatggaaaaa cgccagcaac gcggcctttt 4620
tacggttcct ggccttttgc tggccttttg ctcacatgtt ctttcctgcg ttatcccctg 4680
attctgtgga taaccgtatt accgccatgc at 4712

## Description

### Field of the invention

The present invention relates to improved methods for nucleic acid analysis. In particular, the present invention provides improved methods for carrying out nucleic acid analysis using modified nucleotides.

### Background of the invention

One particularly important application for probes including a reporter is their use in nucleic acid amplification reactions, such as polymerase chain reaction (PCR), to detect the presence and amplification of a target nucleic acid sequence.

As example of such techniques, U.S. Pat. No. 5,210,015 proposed fluorescence-based approaches to provide real time measurements of amplification products during PCR. Such approaches have either employed intercalating dyes (such as ethydium bromide) to indicate the amount of double-stranded DNA present, or they have employed probes containing fluorescence-quenchers pairs (also referred to as the "TAQ-Man" approach), where the probe is cleaved during amplification to release a fluorescent molecule whose concentration is proportional to the amount of double-stranded DNA present. During the amplification, the probe is digested by the nuclease activity of a polymerase when hybridized to the target sequence to cause the fluorescence molecule to be separated from the quencher molecule, thereby causing fluorescence from the reporter to appear.

Recently, the development and use of modified nucleotides has expanded the range of nucleic acid analysis techniques. For example, the modified non-natural nucleotides of ERAGEN CORPORATION expand the alphabet of bases that may be used in nucleic acid technologies (See e.g., U.S. Pat. Nos. 5,216,141, 5,432,272, 5,958,702, 5,965,364, 6,001,983, 6,037,120, 6,140,496, 6,627,456, and 6,617,106, and patent application Ser. Nos. 09/993,757, 60/205,712, 60/240,398, 60/282,831, 60/240,397, 60/252,783, and 60/253,382, each of which is incorporated herein in its entirety).

Modified nucleotides also find use in nucleic acid amplification reactions such as the polymerase chain reaction (See, e.g., U.S. Pat. No No 6,977,161 and patent application Ser. No. 09/861,292, published as Publication No. 2002/0150900, each of which is incorporated herein by reference in its entirety).

Nucleic acid methods, such as the GENECODE methods of ERAGEN CORPORATION employ complementary non-naturally occurring nucleotides such as iso-G and iso-C to expand the alphabet of the genetic code for nucleic acid analysis. In these methods, a non-naturally occurring nucleotide is present in a primer. When the primer is extended in, for example, a polymerase chain reaction, the non-natural base becomes incorporated into one strand of the amplification products. When this strand is used as a template in a subsequent amplification step, ERAGEN reports that the natural bases will not incorporate across from the non-natural base. This failure to incorporate is used as a point of discrimination in some GENECODE methods (e.g., by allowing only labelled, complementary non-natural bases to be added).

### Summary of the invention

The present invention relates to a method for detecting at least one target nucleic acid X in a sample, comprising the steps of:
a) contacting said sample with:
   - a polymerase,
   - natural dNTPs,
   - at least one oligonucleotide probe comprising a first non-natural nucleotide located at its 5' end, and a first label;
   - at least one couple of two oligonucleotide primers complementary to 5' and 3' nucleotidic regions respectively of said at least one target nucleic acid X, and
   - at least one reporter comprising at least a second label coupled to a second non-natural nucleotide, which second non-natural nucleotide is complementary to said first non-natural nucleotide,
b) conducting a polymerase chain reaction under conditions wherein said at least one target nucleic acid X, if present in the sample, is amplified using said couple of primers and said oligonucleotide probe for generating oligonucleotide amplification product having (i) a double-stranded region and (ii) a single-stranded region that comprises the first non-natural nucleotide,
c) incorporating the second non-natural nucleotide of the reporter into the oligonucleotide amplification product opposite to the first non natural nucleotide of the single stranded region,
d) detecting the incorporation of the reporter
   **characterised in that**
i) one of said primers of said at least one couple of primers comprises a first oligonucleotide tag sequence A in its 5' region; and
ii) said at least one oligonucleotide probe consists in said first oligonucleotide tag sequence A, said first non-natural nucleotide located at its 5' end, and said first label.

The present invention also relates to a kit for detecting at least one target nucleic acid X in a sample comprising:
a) a polymerase;
b) natural dNTPs;
c) at least one oligonucleotide probe comprising a first non-natural nucleotide located at its 5' end, and a first label;
d) at least one couple of two oligonucleotide primers complementary to 5' and 3' nucleotidic regions respectively of said at least one target nucleic acid X, and
e) at least one reporter comprising at least a second label coupled to a second non-natural nucleotide, which second non-natural nucleotide is complementary to said first non-natural nucleotide,
   **characterised in that**
i) one of said primers of said at least one couple of primers comprises a first oligonucleotide tag sequence A in its 5' region; and
ii) said at least one oligonucleotide probe consists in said first oligonucleotide tag sequence A, said first non-natural nucleotide located at its 5' end, and said first label.

### Figures and Brief description of the figures

The figure 1 shows the amplification profile of a target sequence diluted at four different concentrations with qPCR by the method of the invention.

The figure 2 shows the logarithmic representation of the target initial quantity diluted at four different concentrations, as a function of the Ct (also called standard curve) extrapolated from amplification profiles presented in figure 1.

The figures 3A and 3B show the amplification profiles of a first (Fig. 3A) and a second (Fig. 3B) target sequences diluted at four different concentrations with qPCR multiplex reaction by the method of the invention.

The figures 4A and 4B show the logarithmic representation of targets initial quantity diluted at four different concentrations, as a function of the Ct extrapolated from amplification profiles presented in figures 3A and 3B respectively.

### Detailed description of the invention

In a first aspect, the present invention relates to a method for detecting at least one target nucleic acid X in a sample, comprising the steps of:
a) contacting said sample with:
   - a polymerase,
   - natural dNTPs,
   - at least one oligonucleotide probe comprising a first non-natural nucleotide located at its 5' end, and a first label;
   - at least one couple of two oligonucleotide primers complementary to 5' and 3' nucleotidic regions respectively of said at least one target nucleic acid X, and
   - at least one reporter comprising at least a second label coupled to a second non-natural nucleotide, which second non-natural nucleotide is complementary to said first non-natural nucleotide,
b) conducting a polymerase chain reaction under conditions wherein said at least one target nucleic acid X, if present in the sample, is amplified using said couple of primers and said oligonucleotide probe for generating oligonucleotide amplification product having (i) a double-stranded region and (ii) a single-stranded region that comprises the first non-natural nucleotide,
c) incorporating the second non-natural nucleotide of the reporter into the oligonucleotide amplification product opposite to the first non natural nucleotide of the single stranded region,
d) detecting the incorporation of the reporter
   **characterised in that**
i) one of said primers of said at least one couple of primers comprises a first oligonucleotide tag sequence A in its 5' region; and
ii) said at least one oligonucleotide probe consists in said first oligonucleotide tag sequence A, said first non-natural nucleotide located at its 5' end, and said first label.

Methods for detecting a target nucleic acid in a sample are well known from the man skilled in the art. For example, such methods include target nucleic acid amplification assays such as polymerase chain reaction (PCR), and more specifically quantitative PCR (qPCR) like TaqMAN®, SYBRGREEN, or PLEXOR^{™}.

The present invention provides a new method to detect a target nucleic acid in a sample, using basically 1) a couple of oligonucleotide primers, and 2) a probe that is composed of an oligonucleotide tag sequence, a non-natural nucleotide and a label. Only the oligonucleotide primers are specific of the target nucleic acid sequence and, on the contrary, the oligonucleotide probe is not.

Among all of the known methods for detecting a target nucleic acid in a sample, the present invention uses some characteristics of the PLEXOR^{™} technology.

The PLEXOR^{™} system is a quantitative PCR method using primers containing modified nucleotides (US Pat. No 7,282,333). PLEXOR^{™} technology takes advantage of the highly specific interaction between two modified nucleotides for qPCR analysis. These two nucleotides form a unique base pair when incorporated in double-stranded DNA and pair only with each other. In PLEXOR^{™} reactions, one PCR primer is synthesized with a modified non-natural nucleotide and a label at the 5'end. In preferred embodiment, the non-natural nucleotide is 5'méthylisocytosine (iso-C) and the label is a fluorescent dye. The second PCR primer is unlabeled. A non-natural nucleotide, complementary to the one of the primer, is coupled with a second label and included in the reaction mix. During amplification, this second non-natural nucleotide is preferentially incorporated at the position complementary to the non-natural nucleotide of the primer. Preferably, this second non-natural nucleotide is isoguanine (iso-G) and it is coupled with a quencher. Incorporation of this second label in close proximity to the label carried by the primer effectively modifies the detectable signal of the label. More preferably, the incorporation of the quencher coupled with iso-dG in close proximity to the fluorescent dye of the primer decreases the fluorescent signal. Using PLEXOR^{™} technology, the accumulation of amplification products results in a reduction in label fluorescence that is proportional to the quantity of the input DNA template. Real-time instrumentation, which couples fluorescence detection and thermal cycling, measures the change of signal at every cycle.

Hence, PLEXOR^{™} system uses one primer specific for particular nucleotide sequence of interest, that also contains non-naturally occurring nucleotide, and that also carries a label. Accordingly, one should use and synthesize such distinct modified primer for each nucleotidic sequence to amplify. As the chemical incorporation of modified nucleotide and label into primers is expansive, each amplification is rendered expansive, and the costs increase considerably when multiplex amplifications are considered.

Among all the known technologies, methods and compositions that reduce the cost of the analysis without affecting assay are needed.

In the present invention, the specificity to the target nucleic acid is carried only by the couple of oligonucleotide primers. Since chemical production of such basic oligonucleotides is now currently proposed by a lot of companies, obtaining such couple of primers is nowadays not expansive at all. The oligonucleotide probe used in the present invention is composed by an oligonucleotide tag sequence, a non-natural nucleotide and a label. As the chemical production of a non-natural nucleotide and its incorporation in a natural oligonucleotide may be costly, the production of the oligonucleotide probe is the critical step of the technology in terms of costs. However, in the present invention, the oligonucleotide probe is not dedicated to one particular target nucleic acid but rather to a common tag sequence carried by one of the primers. Hence, one user may re-use such an oligonucleotide probe in several reactions provided that the oligonucleotide primer has been designed in order to comprise the same tag sequence. Moreover, and unexpectedly, the use of such a probe does not affect the quantitative aspect of the detection of a target sequence as established by the inventors. Finally, the application of such a new technology is particularly interesting in laboratories that have several thematic and genes of interest: each team could buy, at low cost, couples of oligonucleotide primers, and one batch of oligonucleotide probe may be used by all the users independently of their gene of interest.

Consequently, the present invention provides a new method to detect in a quantitative manner a target nucleic acid, as sensitive as the PLEXOR^{™} technology commonly used, but cheaper.

The term "sample" in the present specification and claims is used in its broadest sense as any element containing nucleic acid, such as DNA or RNA. On the one hand it is meant to include a specimen or culture (e.g., microbiological cultures). On the other hand, it is meant to include both biological and environmental samples. A sample may include a specimen of synthetic origin. Biological samples may be animal, including human, fluid, solid (e.g., stool) or tissue, as well as liquid and solid food and feed products and ingredients such as dairy items, vegetables, meat and meat by-products, and waste. Biological samples may be obtained from all of the various families of domestic animals, as well as feral or wild animals, including, but not limited to, such animals as ungulates, bear, fish, lagomorphs, rodents, etc. Environmental samples include environmental material such as surface matter, soil, water and industrial samples, as well as samples obtained from food and dairy processing instruments, apparatus, equipment, utensils, disposable and non-disposable items. These examples are not to be construed as limiting the sample types applicable to the present invention.

The term "nucleic acid" as used herein refers to oligonucleotide, polynucleotide and fragments or portions thereof, preferably to DNA or RNA of genomic or synthetic origin which may be single or double stranded.

As used herein, a "target nucleic acid" refers to a specific nucleic acid of interest. Such nucleic acid of interest corresponds by example to a preRNA or to a RNA coding for a specific protein, to a specific gene, to a cDNA, to a non-genic sequence or to a specific tRNA, rRNA, miRNA, or smRNA.

As an example, such sequence of interest corresponds to dishevelled 3 gene (SEQ ID NO:11) or Kanamycin (SEQ ID NO:12).

The term "oligonucleotide" as used herein refers to RNA or DNA, preferably to DNA, which oligonucleotide may be generated in any manner, including chemical synthesis, DNA replication, reverse transcription, PCR, or a combination thereof.

The term "oligonucleotide primer" refers to an oligonucleotide sequence that is capable of acting as a point of initiation of synthesis when placed under conditions in which primer extension is initiated by polymerase. An "oligonucleotide primer" may occur naturally, as in a purified restriction digest or may be produced synthetically. A primer is selected to be complementary to a strand of the target nucleic acid. A primer must be sufficiently complementary to hybridize with the target nucleic acid strand for primer elongation to occur. A primer sequence needs not to reflect the exact sequence of the target nucleic acid strand. Non-complementary bases or longer sequences can be interspersed into the primer, provided that the primer sequence has sufficient complementarity with the sequence of the target nucleic acid to hybridize and thereby form a nucleic acid / primer complex for synthesis of the extension product of the primer. A primer is single-stranded. In the present invention, primers are always present by couple, and flank the target nucleic acid. Each primer of said couple is complementary to 5' and 3' nucleotidic regions respectively of a specific target nucleic acid.

Each primer of said couple contains at least 10 consecutives nucleotides that are complementary to the target nucleic acid, preferably at least 12 or 15 consecutives nucleotides, and more preferably between 15 and 30 consecutives nucleotides that are complementary to the target nucleic acid.

In the present invention, each primer of said couple is characterised by a melting temperature (TM) of about 50 to 65°C, preferably of 58 to 62°C, and a G/C content of about 40 to 55%, preferably of 45 to 50%.

The concentration of primers to be used in the present method can be simply determined by the skilled person. As specified previously, one primer in said couple of primer contains in its 5' region a first tag sequence which primer has a limiting concentration. Said primer limiting concentration is equal or less than 300nM, preferably equal or less than 150nM, and more preferably equal or less than 100nM.

The other primer of said couple (i.e., the primer with no tag sequence), such as the other components added to the sample at step a) have an excess concentration.

Said primer excess concentration can be simply determined by the skilled person and, by example, is equal or greater than 10 fold more concentrated as compared to the primer having a limiting concentration, preferably equal or greater than 5 fold, and more preferably equal or greater than 1,5 fold more concentrated as compared to the primer having a limiting concentration.

As used herein, the term "Tm" is used in reference to the "melting temperature." The melting temperature is the temperature at which a population of double-stranded nucleic acid molecules becomes half dissociated into single strands. Several equations for calculating the Tm of nucleic acids are well known in the art. As indicated by standard references, a simple estimate of the Tm value may be calculated by the equation: Tm=81.5+0.41(% G+C), when a nucleic acid is in aqueous solution at 1 M NaCl (*See* e.g., Anderson and Young, Quantitative Filter Hybridization, in Nucleic Acid Hybridization (1985). Other references (e.g., Allawi, H. T. & SantaLucia, J., Jr. Thermodynamics and NMR of internal G.T mismatches in DNA. Biochemistry 36, 10581-94 (1997) include more sophisticated computations which take structural and environmental, as well as sequence characteristics into account for the calculation of Tm.

In the conditions of a PCR, the use of said couple of primers should induce the formation of "oligonucleotide amplification product" from the target nucleic acid, whose length is less than 1000 base pairs, preferably less than 500 base pairs, and more preferably less than 300 base pairs.

As used herein, the terms "complementary" or "complementarity" are used in reference to polynucleotides (i.e., a sequence of nucleotides such as an oligonucleotide or a target nucleic acid) related by the base-pairing rules. For example, the sequence "5'-A-G-T-3"' is complementary to the sequence "3'-T-C-A-5"'; said sequence "3'-T-C-A-5'" is the complement of the sequence "5'-A-G-T-3"'. Complementarity may be "partial," in which only some of the nucleic acids' bases are matched according to the base pairing rules. Or there may be "complete" or "total" complementarity between the nucleic acids. Either terms may also be used in reference to individual nucleotides, especially within the context of polynucleotides. For example, a particular nucleotide within an oligonucleotide may be noted for its complementarity, or lack thereof, to a nucleotide within another nucleic acid strand, in contrast or comparison to the complementarity between the rest of the oligonucleotide and the nucleic acid strand.

The term "complement" of a nucleic acid reference sequence as used herein refers to an oligonucleotide which is complementary to said nucleic acid sequence according to base-pairing rules. In the nucleic acid complement sequence, each base can pair with the corresponding base on the nucleic acid reference sequence in the antiparallel association of nucleic acids.

Advantageously, a sequence complementary to a target nucleic acid corresponds to a sequence having more than 80 % of identity with the complement of said target nucleic acid sequence, preferably more than 95 %, and more preferably more than 99 %.

As used herein, "percentage of identity" between two oligonucleotides sequences, means the percentage of identical nucleotides, between the two sequences to be compared, obtained with the best alignment of said sequences, this percentage being purely statistical and the differences between these two sequences being randomly spread over the nucleotide sequences. As used herein, " best alignment" or "optimal alignment", means the alignment for which the determined percentage of identity (see below) is the highest. Sequences comparison between two oligonucleotide sequences are usually realized by comparing these sequences that have been previously align according to the best alignment; this comparison is realized on segments of comparison in order to identify and compared the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developed by NEDDLEMAN and WUNSCH (J. Mol. Biol., vol.48, p:443, 1970), by using the method of similarities developed by PEARSON and LIPMAN (Proc. Natl. Acad. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p:1792, 2004). To get the best local alignment, one can preferably used BLAST software. The identity percentage between two oligonucleotide sequences is determined by comparing these two sequences optimally aligned, the oligonucleotide sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

As used herein, the term "hybridization" is used in reference to the pairing of complementary nucleic acids. Hybridization and the strength of hybridization (i.e., the strength of the association between the nucleic acids) is influenced by such factors as the degree of complementary between the nucleic acids, stringency of the conditions involved, and the Tm of the formed hybrid. "Hybridization" methods involve the annealing of one nucleic acid to another, complementary nucleic acid, i.e., a nucleic acid having a complementary nucleotide sequence. The ability of two polymers of nucleic acid containing complementary sequences to find each other and anneal through base pairing interaction is a well-recognized phenomenon. The initial observations of the "hybridization" process by Marmur and Lane, Proc. Natl. Acad. Sci. USA 46:453 (1960) and Doty et al., Proc. Natl. Acad. Sci. USA 46:461 (1960) have been followed by the refinement of this process into an essential tool of modem biology.

As used herein, a "nucleotide tag sequence" is an oligonucleotide sequence added at the 5' end of one oligonucleotide of the couple of primers used for amplification. This "nucleotide tag sequence" is not complementary to the target nucleic acid(s) or to its (their) complement(s), and preferably to any nucleotide sequence present in the sample. Therefore, a nucleotide tag sequence is not capable of hybridization with the target nucleic acid(s), and preferably with any nucleotide sequence present in the sample.

As used herein, a "nucleotide tag sequence" is single-stranded, and is equal or more than 10 nucleotides in length, preferably equal or more than 12 nucleotides in length, and more preferably equal or more than 15 nucleotides in length.

Preferably said tag sequence has a melting temperature of about 50 to 65°C, preferably of 58 to 63°C, and a G/C content of about 40 to 55%, preferably less than 60 %, and even more preferably of 45 to 50%..

Advantageously, said nucleotide tag sequence has less than 50 % of identity with the target nucleic acid(s) sequence(s) or its(their) complement(s), preferably less than 30 %, as an example less than 20 %, and more preferably less than 10 % of identity with the target nucleic acid(s) or its(their) complement(s).

More advantageously, said nucleotide tag sequence has less than 50 % of identity with any nucleic acid sequence from the sample, preferably less than 30 %, as an example less than 20 %, and more preferably less than 10 % of identity with any nucleic acid sequence from the sample.

As an example, said oligonucleotide tag sequence is selected from the group comprising GCTCGTTACGTGGTTGAGAC (SEQ ID NO:1), CACTTAGCACCACGGACA (SEQ ID NO: 2), CGTTGTAACGCCCCAGCAC (SEQ ID NO: 3), GCCTCTTCCGGTTACCGTGT (SEQ ID NO: 4), AATTAACCCTCACTAAAGGG (SEQ ID NO:5), and CTATAGTGAGTCGTATTA (SEQ ID NO:6).

As used herein, the terms "purified" or "substantially purified" refer to molecules, either nucleic or amino acid sequences, that are removed from their natural environment, isolated or separated, and are at least 60% free, preferably 75% free, and most preferably 90% free from other components with which they are naturally associated. An "isolated polynucleotide" or "isolated oligonucleotide" is therefore a substantially purified polynucleotide.

As used herein, a "polymerase" is an enzyme, preferably thermostable, whose central function is associated with polymers of nucleic acids such as RNA and DNA. The primary function of a polymerase is the polymerization of new DNA or RNA against an existing DNA or RNA strand in the processes of replication and transcription. In association with a cluster of other enzymes and proteins, they take nucleotides from solution, and catalyses the synthesis of a polynucleotide sequence from a nucleotide template strand using base-pairing interactions.

The term "thermostable" refers to a polymerase which is functional or active (i.e., can perform catalysis) at an elevated temperature, i.e., at about 55°C or higher.

The term "natural" as used herein refers to the most common nucleotides components of DNA and RNA nucleic acids, i.e., A, C, G, T and U nucleotidesIn the present invention, "natural dNTPs" refers to naturally existing deoxyribonucleotides tri-phosphates such as deoxyriboAdenosine tri-phosphates, deoxyriboCytosine tri-phosphates, deoxyriboGuanidine tri-phosphates, or deoxyriboThymidine tri-phosphates.

The term "non-natural" or "non-naturally occurring" as used herein refers to nucleotides other than said natural nucleotides. Such non-natural nucleotides include modified natural nucleotides and non-naturally occurring nucleotides, including but not limited to analogs that have altered stacking interactions such as 7-deaza purines (i.e., 7-deaza-dATP and 7-deaza-dGTP); nucleotide analogs with alternative hydrogen bonding configurations (e.g., such as iso-C and iso-G and other non-standard base pairs described in U.S. Pat. No. 6,001,983 to S. Benner, and the selectively binding nucleotide analogs described in U.S. Pat. No. 5,912,340 to Igor V. Kutyavin, et al.); non-hydrogen bonding analogs (e.g., non-polar, aromatic nucleoside analogs such as 2,4-difluorotoluene, described by B. A. Schweitzer and E. T. Kool, J. Org. Chem., 1994, 59, 7238-7242, B. A. Schweitzer and E. T. Kool, J. Am. Chem. Soc., 1995, 117, 1863-1872); "universal" nucleotides such as 5-nitroindole and 3-nitropyrrole; and universal purines and pyrimidines (such as "K" and "P" nucleotides, respectively; P. Kong, et al., Nucleic Acids Res., 1989, 17, 10373-10383, P. Kong et al., Nucleic Acids Res., 1992, 20, 5149-5152). Nucleotide analogs include modified forms of deoxyribonucleotides as well as ribonucleotides. "Non-natural" and "non-naturally occurring" nucleotides are specifically not limited to such nucleotides as are never found in nature. Natural processes such as nucleic acid damage can give rise to "natural" occurrence of nucleotides that are nonetheless not generally considered to be part of the set of "natural" nucleotides as defined herein. For example, iso-G can be found in oxidatively damaged DNA. Such non-natural nucleotides and their behaviors in replication and other nucleic acid syntheses have been extensively studied in contexts such as DNA damage studies, although the compounds are sometimes described using different nomenclature. For example, the ribonucleoside comprising the isoguanosine nucleotides has been referred to in the literature variously as: iG; isoG; iso-G; isoguanosine; 2-hydroxyadenine; 2-oxoadenine; 2-hydroxy A; and 2-OH-A. The deoxyribonucleoside comprising the isoguanosine nucleotides has been referred to variously as: iG; isoG; iso dG; deoxyiso-G; deoxyisoguanosine; 2-hydroxydeoxyadenosine; 2-hydroxy dA; and 2-OH-Ade.

In the invention, at least two distinct non-natural nucleotides are used. Said non-natural nucleotides are **characterised in that** they can pair only with each other. In other terms, they are complementary with each other, and not with any one of natural nucleotides present in the amplification mix.

In a preferred embodiment, iso-G and iso-C will be used as non-natural complementary nucleotides.

In a more preferred embodiment, these non-natural nucleotides have been modified to impair further extension when incorporated in the oligonucleotide amplification product. Such modified non-natural nucleotides include nucleosides, i.e. nucleotides missing the final phosphorylated moiety on the ribose. Such nucleosides are still complementary.

The term "label" as used herein refers to any atom or molecule that can be used to provide a detectable (preferably quantifiable) effect, and that can be attached to a nucleic acid. Labels include but are not limited to dyes; radiolabels such as ³²P; binding moieties such as biotin; haptens such as digoxygenin; luminogenic, phosphorescent or fluorogenic moieties; mass tags; and fluorochromes alone or in combination with quenchers that can suppress or shift emission spectra by fluorescence resonance energy transfer (FRET).

Preferably, said labels are fluorochromes alone or in combination with quenchers that can suppress or shift emission spectra by fluorescence resonance energy transfer (FRET).

Said labels may provide signals detectable by fluorescence, radioactivity, colorimetry, gravimetry, X-ray diffraction or absorption, magnetism, enzymatic activity, characteristics of mass or behavior affected by mass (e.g., MALDI time-of-flight mass spectrometry), and the like, preferably by fluorescence. A label may be a charged moiety (positive or negative charge) or alternatively, may be charge neutral. Labels can include or consist of nucleic acid or protein sequence, so long as the sequence comprising the label is detectable.

The interaction between two labels may produce a detectable effect. The interaction is not limited to any particular nature of interaction. The interaction of the labels may be *via* direct contact, e.g., a covalent or non-covalent contact between two moieties (e.g., a protein-protein contact, or collisional energy transfer between proximal moieties); it may comprise resonance energy transfer (e.g., between one or more dyes, or between a dye and a quencher moieties); it may comprise a diffusion effect, e.g., wherein the product from a reaction occurring at the site of one label diffuses to the site of another label to create a detectable effect.

In the present invention, the labels used are preferably combinations of fluorochrome/quencher couples.

Suitable fluorochromes include, for example:
- fluorescein and derivatives, like hexachloro-fluorescein, tetrachloro-fluorescein, carboxyfluorescein (TAMRA), CAL FLUOR@ (CAL Fluor Green 520, CAL FLUOR Gold 540, CAL FLUOR ORANGE 560, CAL FLUOR RED 590, CAL FLUOR RED 635 available from BIOSEARCH TECHNOLOGIES), succinimidyl ester of carboxyfluorescein (succinimidyl ester of 6-carboxy-2',4,4',5',7,7'-hexachlorofluorescein (HEX^{™}) or succinimidyl ester of 6-carboxy-4',5'-dichloro-2',7'dimethoxyfluorescein (JOE^{™}));
- Rhodamine and derivatives, like 5- or 6-carboxy-X-rhodamine (ROX), N,N,N',N'-tetramethyl-6-carboxyrhodamine;
- Cyanine and derivatives like Cy3, Cy3.5, Cy5, Cy5.5;
- BODIPY® chromophores like 4,4-difluoro-5,7-diphenyl-4-bora-3a,4a-diaza-s-indacene-3-propionic acid, 4,4-difluoro-5,p-methoxyphenyl-4-bora-3a,4a-diaza-s-indacene-3-propionic acid, 4,4-difluoro-5-styryl-4-bora-3a,4-adiaz-a-S-indacene-propionic acid, 4,4-difluoro-5,7-diphenyl-4-bora-3a,4a-diaza-s-indacene-3-propionic acid, 4,4-difluoro-5,p-ethoxyphenyl-4-bora-3a,4a-diaza-s-indacene 3-propionic acid and 4,4-difluoro-5-styryl-4-bora-3a,4a-diaza-S-indacene-propionic acid;
- Texas Red® and derivatives;
- Pyrenetrisulfonic acid like APTS, HPTS (CASCADE BLUE®); and
- Eosin and derivatives.

Suitable quenchers include, for example, Dabcyl (4-((4-(dimethylamino)phenyl)azo)benzoic acid, succinimidyl ester), QSY® dyes like QSY7 (9-[2-[[4-[[(2,5-dioxo-1-pyrrolidinyl) oxy]carbonyl]-1- piperidinyl]sulfonyl]phenyl]-3,6- bis).

In some embodiments, fluorochromes can also be used as a quencher (e.g., if they absorb the emitted light of another dye).

Advantageously, said fluorochrome is selected in the group comprising fluorescein and derivatives like hexachloro-fluorescein, tetrachloro-fluorescein, carboxyfluorescein (TAMRA), CAL FLUOR@ (CAL Fluor Green 520, CAL FLUOR Gold 540, CAL FLUOR ORANGE 560, CAL FLUOR RED 590, CAL FLUOR RED 635 available from BIOSEARCH TECHNOLOGIES), succinimidyl ester of carboxyfluorescein (succinimidyl ester of 6-carboxy-2',4,4',5',7,7'-hexachlorofluorescein (HEX^{™}) or succinimidyl ester of 6-carboxy-4',5'-dichloro-2',7'dimethoxyfluorescein (JOE^{™})) and said quencher is dabcyl, which absorbs between 400 and 525 nm (maximum at 475 nm).

Because mononucleotides are reacted to make oligonucleotides in a manner such that the 5' phosphate of one mononucleotide pentose ring is attached to the 3' oxygen of its neighbor in one direction via a phosphodiester linkage, an end of an oligonucleotide is referred to as the "5' end" if its 5' phosphate is not linked to the 3' oxygen of another mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have 5' and 3' ends.

As used herein, the term "distinct" in reference to signals refers to signals that can be differentiated one from another, e.g., by spectral properties such as fluorescence emission wavelength, color, absorbance, mass, size, fluorescence polarization properties, charge, etc., or by capability of interaction with another moiety, such as with a chemical reagent, an enzyme, an antibody, etc.

The present invention also refers to an "oligonucleotide probe" that comprises a non-natural nucleotide located at its 5' end, a label and a nucleotide tag sequence. Preferably, said non-natural nucleotide and said label are coupled.

As used herein, two entities are "coupled" when they are linked covalently or by strong hydrogen bounds.

Advantageously, said tag sequence and said non-natural nucleotide are coupled, the non-natural nucleotide being located at the 5' end of the tag sequence.

More advantageously, the oligonucleotide probe consists in an oligonucleotide tag sequence, a non-natural nucleotide at the 5' end, and a label. Preferably, the oligonucleotide probe consists in an oligonucleotide tag sequence coupled to a non-natural nucleotide at its 5' end, and a label coupled to said non-natural nucleotide.

Methods for detecting the incorporation of the reporter are well known from the skilled person and depend on the used labels.

In some embodiments, the accumulation of oligonucleotide amplification product may be detected by quenching the signal of the label incorporated in said product by site-specific incorporation of a quencher near said label.

Specifically, the hydridization between the non-natural nucleotide carrying the quencher and the non-natural nucleotide carrying the fluorochrome which is incorporated in each oligonucleotide amplification product is measured by a decrease in fluorescence of said fluorochrome.

In an alternative embodiment, fluorescence energy transfer (FRET) can be detected between the label of the probe (donor dye) and the label of the reporter (acceptor dye). Detection of oligonucleotide amplification product can be observed by exciting the donor dye and reading the emission of the incorporated acceptor dye.

Specifically, the hydridization between the non-natural nucleotide carrying the "quencher" (acceptor dye) and the non-natural nucleotide carrying the fluorochrome (donor dye) which is incorporated in each oligonucleotide amplification product is measured by an increase of the fluorescence of the "quencher" (acceptor dye) following the excitation of the fluorochrome (donor dye).

As in the PLEXOR technology, oligonucleotide amplification products containing fluorophores quenched by site-specific incorporation of a quenching compound may be subjected to melt curve analysis, e.g., in an instrument that can monitor fluorescence differences during temperature changes. The change in fluorescence is monitored while gradually increasing the temperature of the amplification reaction products (e.g., at a rate of 0.1.degree. C. per second). The Tm of the intended product (quencher-incorporated PCR product) as well as that of any nonspecific product (e.g., quencher-incorporated products that have been chain-terminated prematurely using the extension-disabled non-natural nucleotides of the present invention, or quencher-incorporated primer/dimers) may thus be determined. If the Tm of the intended product is selected to be substantially higher than the Tm of primer/dimers and the terminated products, the signal generated by the intended product may be specifically observed (and thus the presence or absence, or the quantity of the initial target material may be determined) by taking the fluorescent measurement of the reaction at a temperature above the Tm of the nonspecific products.

Using the methods of the present invention, one is able to conduct techniques with long oligonucleotide amplification product (e.g., greater than 50 nucleotides, greater than 100, greater than 500, greater than 1000, greater than 2000, etc.), without incurring detrimental background signal.

A Multiplex reaction can also be conducted when several target nucleic acids are present in the same sample. In such a situation, one may use the present invention to quantify the initial concentration, relative or absolute, of all the target nucleic acids present in the sample prior the amplification.

In another embodiment, the present invention is thus directed to the detection of at least two target nucleic acids X and Y in a sample, further comprising the steps of:
a) contacting said sample with:
   - at least one couple of two primers complementary to 5' and 3' nucleotidic regions respectively of said at least one target nucleic acid Y, wherein one of said two primers comprises another oligonucleotide tag sequence B in its 5' region; and
   - at least a second oligonucleotidic probe consisting in said second oligonucleotide tag sequence B, a third non-natural nucleotide located at its 5' end, and a third label;
   - at least one reporter comprising at least a forth label coupled to a forth non-natural nucleotide, which forth non-natural nucleotide is complementary to said third non-natural nucleotide of said second oligonucleotidic probe;
b) conducting a polymerase chain reaction under conditions wherein said at least two target nucleic acids X and Y, if presents in the sample, are amplified using said two couples of primers and said two oligonucleotidic probes for generating two oligonucleotidic amplification products having (i) double-stranded regions and (ii) single-stranded regions that comprise the first and third non-natural nucleotides respectively,
c) incorporating the non-natural nucleotides of the two reporters into the two oligonucleotidic amplification products opposite to the first and third non natural nucleotides of the single stranded regions respectively,
d) detecting the incorporation of the two reporters.

Preferably, said third label is a second fluorochrome which is different from the fluorochrome corresponding to the first label, and said forth label is a quencher.

More preferably, said third label is selected in the group comprising fluorescein and derivatives like hexachloro-fluorescein, tetrachloro-fluorescein, carboxyfluorescein (TAMRA), CAL FLUOR@ (CAL Fluor Green 520, CAL FLUOR Gold 540, CAL FLUOR ORANGE 560, CAL FLUOR RED 590, CAL FLUOR RED 635 available from BIOSEARCH TECHNOLOGIES), succinimidyl ester of carboxyfluorescein (succinimidyl ester of 6-carboxy-2',4,4',5',7,7'-hexachlorofluorescein (HEX^{™}) or succinimidyl ester of 6-carboxy-4',5'-dichloro-2',7'dimethoxyfluorescein (JOE^{™})) and said forth label is dabcyl, which absorbs between 400 and 525 nm (maximum at 475 nm).

In fact, and due to the large spectrum of dabcyl absorbance, said quencher can be used simultaneously with several fluorochromes.

Preferably, said third and forth non-natural nucleotides are **characterised in that** they can pair only with each other, and even more preferably iso-G and iso-C are used as non-natural complementary nucleotides.

Another object of the present invention is directed to a kit for detecting at least one target nucleic acid X in a sample comprising:
a) a polymerase;
b) natural dNTPs;
c) at least one oligonucleotide probe comprising a first non-natural nucleotide located at its 5' end, and a first label;
d) at least one couple of two oligonucleotide primers complementary to 5' and 3' nucleotidic regions respectively of said at least one target nucleic acid X, and
e) at least one reporter comprising at least a second label coupled to a second non-natural nucleotide, which second non-natural nucleotide is complementary to said first non-natural nucleotide,
   **characterised in that**
i) one of said primers of said at least one couple of primers comprises a first oligonucleotide tag sequence A in its 5' region; and
ii) said at least one oligonucleotide probe consists in said first oligonucleotide tag sequence A, said first non-natural nucleotide located at its 5' end, and said first label.

Advantageously, said polymerase, natural dNTPs, oligonucleotide probe, oligonucleotide primer, non-natural nucleotide, oligonucleotide tag sequence and labels are as described previously.

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention that are obvious to those skilled in the relevant fields are intended to be within the scope of the following claims.

### EXAMPLES

### 1) quantization of the dishevelled 3 gene with the method of the invention

In order to evaluate the quantitative skill of the method of the invention several amplification experiences have been realized on the dishevelled 3 gene (SEQ ID NO: 11).

### a) Target sequence and template preparation

The target sequence consisted in the plasmid 1 comprising the sequence of the dishevelled 3 gene (SEQ ID NO:11) cloned in the pmaxFP™-Green-N vector (SEQ ID NO:13).

The template was diluted in MOPS/EDTA buffer to a concentration of 5ng/µL, and serial dilutions (i.e., 1000, 100, and 100) of this template were further made in MOPS/EDTA.

### b) Primers and probe

For the method of the invention, the sequences of the primers and of the probe were as follow:
Primer 421 with Tag: 5'-aattaaccctcactaaaggggtggtgaaggaagag-3' (SEQ ID NO: 7)
Primer 422: 5'-tctccgtgtcattgtccag-3' (SEQ ID NO: 8)
Probe 1 (tag): 5'-aattaaccctcactaaaggg- 3' (SEQ ID NO:5)

### c) Reactions set up

Briefly, the PLEXOR^{™} Master Mix and primers were thawed on ice and shocked to mix.

Duplicated reaction mixes containing PLEXOR^{™} Master Mix, primers, and the probe were obtained using the concentrations indicated in table 1, which mixes were added in appropriate wells of an optical-grade PCR plate on ice.

**Table 1**

| **components** | **Method of the invention** |
|---|---|
| PLEXOR^{™} Master Mix, 2X | 12.5 µl |
| Primer 421 2.5 µM | 1 µl |
| Primer 422 2.5 µM | 1.5 µl |
| Probe 2.5 µM | 1.5 µl |
| Template | 2 µl |
| Nuclease-free water | up to 25.00 µl |

### d) Amplification conditions

The plate was then introduced in DNA Engine Opticon 2 Real-Time PCR Detection System (BIORAD).

The amplification program was as follow:
- 96°C 5min
- 96°C 30sec
- 60°C 30sec,
the two last steps are repeated for 40 cycles.

Finally, the generated data were analysed with the PLEXOR^{™} Analysis software.

### e) Results

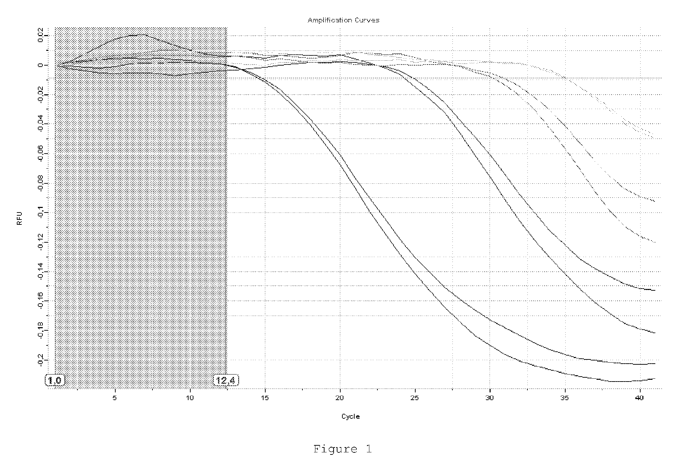

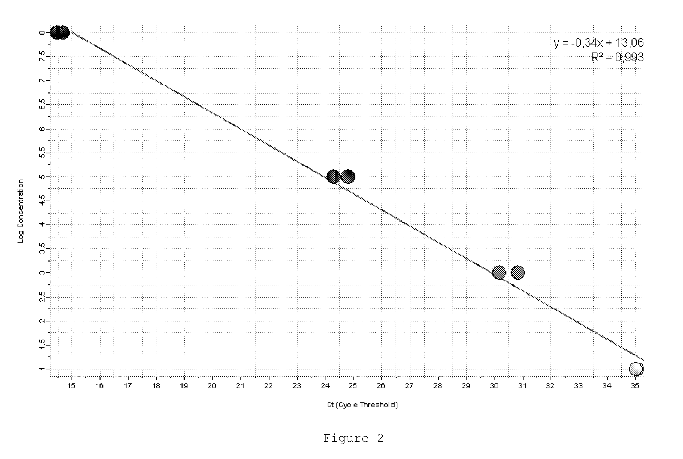

The amplification profiles obtained for the method of the invention is presented in Figure 1. The results established that the amplification curves obtained with the method of the invention (i.e., use of three primers) are proportional to initial quantity of target. This quantitative aspect of profiles is confirmed by standard curves generated from the amplification curve data obtained, which standard curves present the Ct (i.e., cycle threshold) as a function of the logarithm of the target sequence quantity (see Figure 2)

Finally, the method of the invention shows detection sensitivity similar to the one obtained with the PLEXOR^{™} technology.

### 2) Quantization of two target sequences with the method of the invention (multiplex reaction)

### a) Target sequences and template preparation

The target sequence 1 consisted in the plasmid comprising the sequence of the dishevelled 3 gene (SEQ ID NO:11) cloned in the pmaxFP™-Green-N vector (SEQ ID NO:13).

The target sequence 2 consisted in the plasmid comprising the sequence of the Kanamycin gene (SEQ ID NO:12) cloned in the pmaxFP™-Green-N vector (SEQ ID NO:13).

The two targets are shared by the same plasmid.

The templates were diluted in MOPS/EDTA buffer to a concentration of 5ng/µL, and serial dilutions (i.e., 1000, 100, and 100) of these template were further made in MOPS/EDTA.

### b) Primers and probe

For the target sequence 1, the sequences of the primers and of the probe were as follow:
Primer 421 with Tag 1: 5'-aattaaccctcactaaaggggtggtgaaggaagag-3' (SEQ ID NO: 7)
Primer 422: 5'-tctccgtgtcattgtccag-3' (SEQ ID NO: 8)
Probe 1(Tag 1): 5' -aattaaccctcactaaaggg- 3' (SEQ ID NO:5)

For the target sequence 2, the sequences of the primers and of the probe were as follow:
Primer 3 with Tag 2: 5'-ctatagtgagtcgtattagtgacaacgtcgagcacag-3' (SEQ ID NO:9)
Primer 4: 5'-tgaatgaactgcaggacgag-3' (SEQ ID NO:10)
Probe 2 (Tag 2): 5' -ctatagtgagtcgtatta- 3' (SEQ ID NO:6)

### c) Reactions set up

Duplicated reaction mixes containing PLEXOR^{™} Master Mix, primers, and the probe were obtained using the concentrations indicated in table 1, which mixes were added in appropriate wells of an optical-grade PCR plate on ice.

**Table 2**

| **components** | **Quantity** |
|---|---|
| PLEXOR^{™} Master Mix, 2X | 12.5 µl |
| Primer 421 2.5 µM | 1 µl |
| Primer 422 2.5 µM | 1.5 µl |
| Probe 1 2.5 µM | 1.5 µl |
| Template 1 | 2 µl |
| Primer 3 2.5 µM | 1 µl |
| Primer 4 2.5 µM | 1.5 µl |
| Probe 2 2.5 µM | 1.5 µl |
| Template 2 | 2 µl |
| Nuclease-free water | up to 25.00 µl |

### d) Amplification conditions

The plate was then introduced in DNA Engine Opticon 2 Real-Time PCR Detection System (BIORAD).

The amplification program was as follow:
- 96°C 5min
- 96°C 30sec
- 60°C 30sec,
the two last steps are repeated for 40 cycles.

Finally, the generated data were analysed with the PLEXOR^{™} Analysis software.

### e) Results

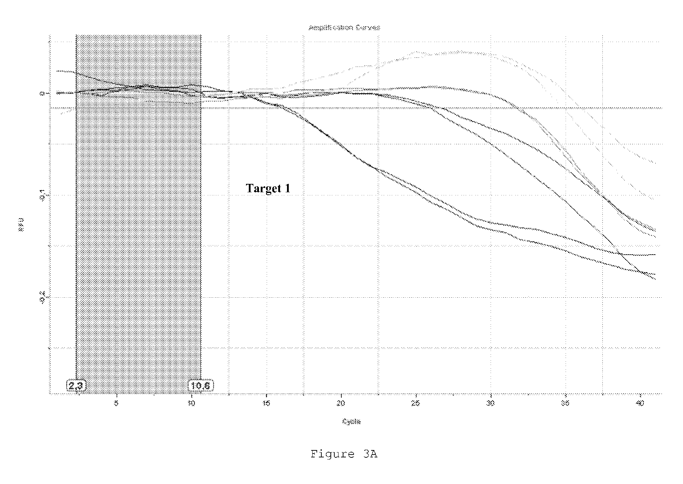

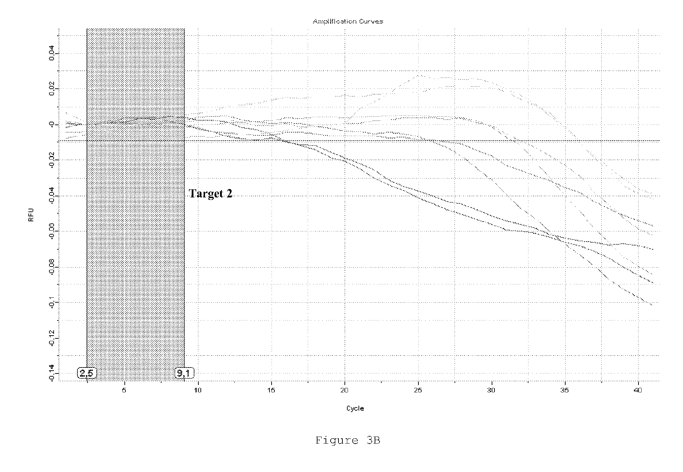

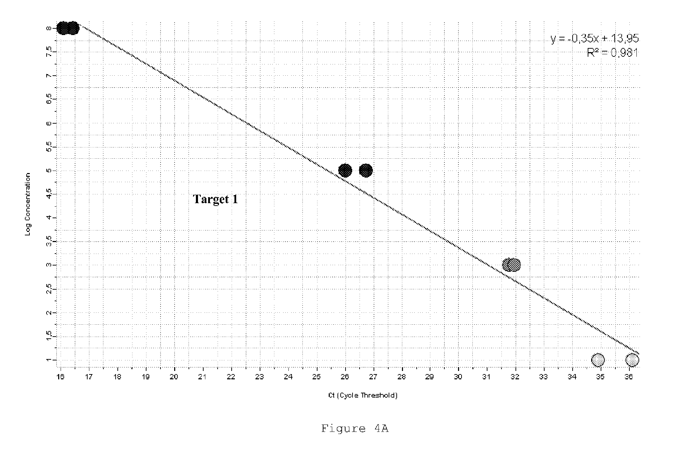

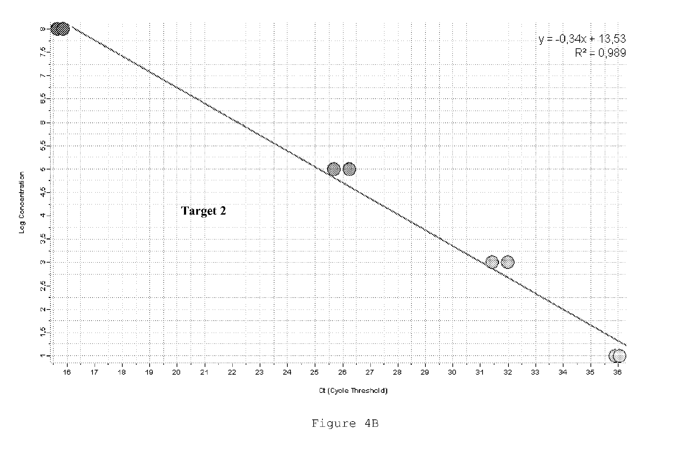

The amplification profiles obtained for the method of the invention is presented in figures 3A and 3B. The results established that the amplification curves obtained with the method of the invention are still proportional to initial quantity of target sequences in a multiplex reaction. The standard curves generated from the amplification curve data obtained for the target sequences 1 & 2 are presented in figures 4A and 4B, which standard curves present the Ct (i.e., cycle threshold) as a function of the logarithm of the target sequences quantities. These results established that the method of the invention enable to obtain simultaneously a good detection in a quantitative manner for at least two target sequences.

Finally, the method of the invention allowed qPCR multiplex reactions in a quantitative manner.

## Claims

1. A method for detecting at least one target nucleic acid X in a sample, comprising the steps of:
a) contacting said sample with:
- a polymerase,
- natural dNTPs,
- at least one oligonucleotide probe comprising a first non-natural nucleotide located at its 5' end, and a first label;
- at least one couple of two oligonucleotide primers complementary to 5' and 3' nucleotidic regions respectively of said at least one target nucleic acid X, and
- at least one reporter comprising at least a second label coupled to a second non-natural nucleotide, which second non-natural nucleotide is complementary to said first non-natural nucleotide,
b) conducting a polymerase chain reaction under conditions wherein said at least one target nucleic acid X, if present in the sample, is amplified using said couple of primers and said oligonucleotide probe for generating oligonucleotide amplification product having (i) a double-stranded region and (ii) a single-stranded region that comprises the first non-natural nucleotide,
c) incorporating the second non-natural nucleotide of the reporter into the oligonucleotide amplification product opposite to the first non natural nucleotide of the single stranded region,
d) detecting the incorporation of the reporter
**characterised in that**
i) one of said primers of said at least one couple of primers comprises a first oligonucleotide tag sequence A in its 5' region; and
ii) said at least one oligonucleotide probe consists in said first oligonucleotide tag sequence A, said first non-natural nucleotide located at its 5' end, and said first label.

2. The method of claim 1, wherein the non-natural nucleotides are selected in the group comprising 7-deaza purines, iso-Cytosine (iso-C), iso-Guanosine (iso-G), 2,4 difluorotoluene, 5-nitroindole, and 3-nitropyrrole, K and P nucleotides.

3. The method of claim 2, wherein the first and the second non-natural nucleotides are iso-C and iso-G.

4. The method of any one of claims 1 to 3, wherein the labels are selected in the group comprising dyes, radiolabels such as ³²P, binding moieties such as biotin, haptens such as digoxygenin, luminogenic or phosphorescent moieties, fluorochromes and quenchers.

5. The method of claim 4, wherein the first and the second labels corresponds to a fluorochrome and a quencher.

6. The method of claim 5, wherein said fluorochrome is selected in the group comprising fluorescein and derivatives like hexachloro-fluorescein, tetrachloro-fluorescein, carboxyfluorescein (TAMRA), CAL FLUOR@ (CAL Fluor Green 520, CAL FLUOR Gold 540, CAL FLUOR ORANGE 560, CAL FLUOR RED 590, CAL FLUOR RED 635 available from BIOSEARCH TECHNOLOGIES), succinimidyl ester of carboxyfluorescein (succinimidyl ester of 6-carboxy-2',4,4',5',7,7'-hexachlorofluorescein (HEX^{™}) or succinimidyl ester of 6-carboxy-4',5'-dichloro-2',7'dimethoxyfluorescein (JOE^{™})).

7. The method of claim 5, wherein said quencher is dabcyl.

8. The method of any one of claims 1 to 7, wherein the oligonucleotide probe consists in an oligonucleotide tag sequence coupled to a non-natural nucleotide at its 5'end, and a label coupled to said non-natural nucleotide.

9. The method of any one of claims 1 to 8, wherein the oligonucleotide tag sequence is not complementary to said target nucleic acid sequence or to its complement.

10. The method of claim 9, wherein said oligonucleotide tag sequence is selected in the group comprising GCTCGTTACGTGGTTGAGAC (SEQ ID NO:1), CACTTAGCACCACGGACA (SEQ ID NO:2), CGTTGTAACGCCCCAGCAC (SEQ ID NO:3), GCCTCTTCCGGTTACCGTGT (SEQ ID NO:4), AATTAACCCTCACTAAAGGG (SEQ ID NO:5), and CTATAGTGAGTCGTATTA (SEQ ID NO:6).

11. The method of any one of claims 1 to 10, wherein said method is directed to the detection of at least two target nucleic acids X and Y in a sample, further comprising the steps of:
a) contacting said sample with:
- at least one couple of two primers complementary to 5' and 3' nucleotidic regions respectively of said at least one target nucleic acid Y, wherein one of said two primers comprises another oligonucleotide tag sequence B in its 5' region; and
- at least a second oligonucleotidic probe consisting in said second oligonucleotide tag sequence B, a third non-natural nucleotide located at its 5' end, and a third label;
- at least one reporter comprising at least a forth label coupled to a forth non-natural nucleotide, which forth non-natural nucleotide is complementary to said third non-natural nucleotide of said second oligonucleotidic probe;
b) conducting a polymerase chain reaction under conditions wherein said at least two target nucleic acids X and Y, if presents in the sample, are amplified using said two couples of primers and said two oligonucleotidic probes for generating two oligonucleotidic amplification products having (i) double-stranded regions and (ii) single-stranded regions that comprise the first and third non-natural nucleotides respectively,
c) incorporating the second and forth non-natural nucleotides of the two reporters into the two oligonucleotidic amplification products opposite to the first and third non natural nucleotides of the single stranded regions respectively,
d) detecting the incorporation of the two reporters.

12. The method of claim 11, wherein the first and the second labels corresponds to a fluorochrome and a quencher, and the third and the forth labels also corresponds to a fluorochrome and a quencher.

13. A kit for detecting at least one target nucleic acid X in a sample comprising:
a) a polymerase;
b) natural dNTPs;
c) at least one oligonucleotide probe comprising a first non-natural nucleotide located at its 5' end, and a first label;
d) at least one couple of two oligonucleotide primers complementary to 5' and 3' nucleotidic regions respectively of said at least one target nucleic acid X, and
e) at least one reporter comprising at least a second label coupled to a second non-natural nucleotide, which second non-natural nucleotide is complementary to said first non-natural nucleotide,
**characterised in that**
i) one of said primers of said at least one couple of primers comprises a first oligonucleotide tag sequence A in its 5' region; and
ii) said at least one oligonucleotide probe consists in said first oligonucleotide tag sequence A, said first non-natural nucleotide located at its 5' end, and said first label.
